# EUROPÄISCHE PATENTSCHRIFT

(11) **EP 2 810 671 B1**
(45) Veröffentlichungstag und Bekanntmachung des Hinweises auf die Patenterteilung: **02.01.2019**
(21) Anmeldenummer: 14169453.9
(22) Anmeldetag: 22.05.2014
(51) Int. Cl.: A61M 3/02, B05B 7/24

(54) **Medizinische Sprühvorrichtung mit Überdruckventil und Verfahren zum Erzeugen eines Sprühkegels**
Medical spraying device with pressure relief valve, and method for producing a spray cone
Dispositif de vaporisation médical équipé d'une soupape de surpression et procédé de fabrication d'un atomiseur

(30) Priorität: 06.06.2013 DE 102013210511
(43) Veröffentlichungstag der Anmeldung: 10.12.2014
(73) Patentinhaber: Heraeus Medical GmbH, 61273 Wehrheim (DE)
(72) Erfinder: Vogt, Sebastian, 99092 Erfurt (DE)
(74) Vertreter: Schultheiss & Sterzel Patentanwälte PartG mbB

(56) Entgegenhaltungen:
- CN-Y- 2 474 228
- DE-A1-102011 018 708
- US-A- 4 940 185
- US-A1- 2001 037 095
- US-A1- 2007 163 573

## Beschreibung

Die Erfindung betrifft eine medizinische Sprühvorrichtung zum Ausspülen einer Wunde, insbesondere ein Lavage-System, sowie die Verwendung einer solchen Sprühvorrichtung. Weiterhin ist auch ein Verfahren zum Erzeugen eines Sprühkegels mit einer medizinischen Sprühvorrichtung Gegenstand der Erfindung.
Gegenstand der Erfindung ist somit eine durch Druckgas getriebene medizinische Sprühvorrichtung für die Unfallchirurgie und Orthopädie. Die Sprühvorrichtung kann im Wesentlichen aus Kunststoffen aufgebaut werden und ist bevorzugt für die einmalige Verwendung bestimmt.
Medizinische Sprühvorrichtungen werden im medizinischen Bereich häufig als Lavage-Systeme bezeichnet. Lavage-Systeme werden in der Chirurgie bei Operationen (OPs) in breitem Umfang eingesetzt, um Gewebeareale zu reinigen. Dabei werden häufig physiologische Kochsalzlösung und Ringer-Lösung als Spülflüssigkeiten verwendet. Mit den Lavage-Systemen werden mit den Spülflüssigkeiten Sprühkegel beziehungsweise Sprühstrahlen erzeugt, die auf die zu reinigenden Gewebeareale auftreffen und eine mechanische Reinigungswirkung auf diese Gewebeareale ausüben. Insbesondere bei der Implantation von Gelenkendoprothesen und bei septischen Revisionen sind Lavage-Systeme von wesentlicher Bedeutung (R. M. Sherman et al.: The role of lavage in preventing hemodynamic and blood-gas changes during cemented arthroplasty. J. Bone Joint. Surg. 1983; 65-A: 500-506.; S. J. Breusch et al.: Lavage technique in THA: Jet-lavage Produces Better Cement Penetration Than Syringe-Lavage in the Proximal Femur. J. Arthroplasty. 200; 15(7): 921-927.; R. J. Byrick et al.: High-volume, high pressure pulsatile lavage during cemented arthroplasty. J. Bone Joint Surg. 1989; 81-A: 1331-1336.; J. Christie et al.: Medullary lavage reduces embolic phenomena and cardiopulmonary changes during cemented hemiarthorplasty. J. Bone Joint Surg. 1995; 77-B: 456-459.) Gepulste Lavage-Systeme sind beispielsweise aus der US 4,583,531 A, der US 4,278,078 A und der US 5,542,918 A bekannt. Die DE 10 2011 018 708 A1 offenbart eine Sprühvorrichtung, die mit einer Gaspatrone betrieben werden kann, indem ein Kolbenvibrator angetrieben wird.

Die CN 2474228Y offenbart eine Sprühvorrichtung aufweisend ein Druckgasreservoir verbunden über eine Druckgasleitung mit einem Flüssigkeitsreservoir, so dass die Flüssigkeit von dem auf die Flüssigkeit wirkenden Gasdruck des Druckgasreservoirs durch eine Düse drückbar ist. Dieses Dokument offenbart auch ein Überdruckventil in der Wandung des Flüssigkeitsreservoirs. Die gegenwärtig im Markt befindlichen Lavage-Systeme werden durch Elektromotoren (zum Beispiel InterPulse® Jet lavage der Stryker GmbH & Co. KG) oder durch Druckluft (zum Beispiel PALAVAGE® der Heraeus Medical GmbH) angetrieben. Bei elektrisch angetriebenen Lavage-Systemen muss jedoch immer ein großer Batterieblock oder Akkumulatorblock mitgeführt werden, der naturgemäß nur eine begrenzte Ladungskapazität hat. Batterie- und Akkumulatorblöcke werden im Hinblick auf ihre Umweltfreundlichkeit kritisch diskutiert. Druckluftgetriebene Lavage-Systeme haben den Vorteil, dass Druckluft im Operationssaal häufig unlimitiert zur Verfügung steht und damit beliebig lange Spülflüssigkeit versprüht werden kann, ohne dass die Energiezufuhr begrenzt ist.

Bei den mit Druckluft oder einem anderen komprimierten Gas getriebenen Systemen wird üblicherweise ein Druckgasmotor zum Antrieb eingesetzt. Bei den meisten Druckgasmotoren für Lavage-Systeme handelt es sich um Lamellen-Druckgasmotoren. Der Druckgasmotor erzeugt eine Drehbewegung, die dann in eine oszillierende, lineare Bewegung umgesetzt wird. Die oszillierende, lineare Bewegung wird genutzt, um jeweils kleinen Volumina des Spülmediums einen Impuls zu verleihen. Dabei wird üblicherweise mindestens eine Membran zwischen dem Antrieb und dem Zulauf der Spülflüssigkeit angeordnet, um die Impulse auf die Spülflüssigkeit übertragen zu können. Es entstehen dadurch Sprühstöße bei hohen Pulszahlen von 2000 bis 3000 Impulsen pro Minute. Das bedeutet, dass der Druckgasmotor sehr präzise gefertigt sein muss, um entsprechend hohe Drehzahlen zu tolerieren. Weiterhin muss eine entsprechend stabile Lagerung vorhanden sein. Aus diesen Gründen ist der Druckgasmotor bei üblichen Druckluft-getriebenen Lavage-Systemen das kostenintensivste Bauteil. Es wird deshalb im Allgemeinen der Druckgasmotor in einem Handgriff aus Metall oder anderen langzeitstabilen Materialien angeordnet, so dass dieses Bauteil nach entsprechender Aufbereitung und Sterilisation mehrfach genutzt werden kann.

Nachteilig ist hieran, dass der Aufbau vieler bekannter Lavage-Systeme relativ kompliziert und dadurch kostenaufwendig ist. Durch den Aufbau mit einem Motor besteht immer die Gefahr einer Fehlfunktion des Motors und damit einer Fehlfunktion des Lavage-Systems. Bei einer mehrfachen Anwendung müssen die Lavage-Systeme desinfiziert und vorbereitet werden. Da es bei der Desinfektion zu Fehlern kommen kann, sind eine Verunreinigung der Wunden des Patienten und damit eine komplizierte Infektion nicht auszuschließen. Auch ist die Geräuschentwicklung des Motors im OP-Betrieb störend und für das medizinische Personal belastend.

Die Aufgabe der Erfindung besteht also darin, die Nachteile des Stands der Technik zu überwinden. Insbesondere soll eine möglichst kostengünstig zu fertigende medizinische Sprühvorrichtung bereitgestellt werden, die einen Sprühkegel erzeugt, der zum Debridieren von Wunden geeignet ist.

Die Aufgabe der Erfindung besteht ferner darin, eine einfach zu fertigende medizinische Sprühvorrichtung zu entwickeln, die möglichst einfach aufgebaut ist und die zur einmaligen Verwendung bestimmt sein kann. Der Aufbau der Sprühvorrichtung soll maximal vereinfacht sein und soll aus möglichst wenigen Teilen bestehen. Die Vorrichtung soll möglichst keine Batterien oder Akkumulatoren enthalten. Die Sprühvorrichtung soll weiterhin unabhängig von äußeren Energiequellen ortsunabhängig betrieben werden können. Die zu entwickelnde Sprühvorrichtung soll im Wesentlichen aus kostengünstigen Kunststoffspritzgießteilen gefertigt werden können. Die Vorrichtung soll in der Lage sein, eine medizinische Spülflüssigkeit anzutreiben und somit einen aus Spülflüssigkeitströpfchen gebildeten Strahl beziehungsweise Sprühkegel zu erzeugen, wobei die Spülflüssigkeitströpfchen im Sprühkegel Zufalls-verteilt auftreten sollen. Des Weiteren soll die Vorrichtung möglichst geräuscharm arbeiten.
Die Aufgaben der Erfindung werden gelöst durch eine medizinische Sprühvorrichtung zum Ausspülen einer Wunde, insbesondere Lavage-System, aufweisend ein Flüssigkeitsreservoir für eine medizinische Spülflüssigkeit oder ein Anschluss für ein solches Flüssigkeitsreservoir und ein Druckgasreservoir, wobei das Druckgasreservoir über eine Druckgasleitung mit dem Flüssigkeitsreservoir verbunden oder verbindbar ist, so dass die Spülflüssigkeit von dem auf die Spülflüssigkeit wirkenden Gasdruck des Druckgasreservoirs durch eine Düse drückbar ist, um einen Sprühkegel zu erzeugen, wobei in der Wandung der Druckgasleitung wenigstens ein Überdruckventil angeordnet ist, das die Druckgasleitung in Abhängigkeit von dem Gasdruck aus dem Druckgasreservoir nach außen öffnet und schließt.
Erfindungsgemäß bevorzugt öffnet sich das Überdruckventil ab einem Grenzdruck zwischen 5 bar und 10 bar.
Nur durch die Anordnung eines Überdruckventils kann erreicht werden, dass die medizinische Spülflüssigkeit direkt mit dem Gasdruck aus der Druckgasleitung beaufschlagt werden kann und dadurch die Sprühvorrichtung ohne Motor aufgebaut werden kann, ohne dass es zu gefährlichen Überdrucken in der Sprühvorrichtung kommen kann.
Bei erfindungsgemäßen Spülvorrichtungen kann vorgesehen sein, dass das Flüssigkeitsreservoir über eine Flüssigkeitsleitung mit der Düse verbunden oder verbindbar ist, wobei in der Flüssigkeitsleitung ein manuell betätigbares Ventilelement angeordnet ist, das bevorzugt zur Steuerung des Volumenstroms der Spülflüssigkeit geeignet ist und das besonders bevorzugt mit einem Abzug bedienbar ist.
Hierdurch kann der Sprühkegel aufgrund einer manuellen Bedienung erzeugt werden.

Ferner kann vorgesehen sein, dass das Flüssigkeitsreservoir eine Flasche mit einer medizinischen Spülflüssigkeit ist, die über die Druckgasleitung und/oder über die Flüssigkeitsleitung mit der Sprühvorrichtung verbunden oder verbindbar ist, wobei bevorzugt die Flüssigkeitsleitung und die Druckgasleitung durch die gleiche Öffnung der im Betrieb kopfüber angeordneten Flasche münden.

Bevorzugt ist die Flasche über die Druckgasleitung und über die Flüssigkeitsleitung mit der Sprühvorrichtung verbunden oder verbindbar. Mit dieser Ausgestaltung der Erfindung kann eine separate Flasche mit der Spülflüssigkeit verwendet werden, ohne dass die Spülflüssigkeit zuvor in die Vorrichtung eingefüllt werden muss. Die Flaschen können auch leichter gewechselt werden, wenn mehr als der Inhalt einer Flasche zur Behandlung notwendig ist.

Mit einer bevorzugten Ausgestaltung der Erfindung wird vorgeschlagen, dass das Flüssigkeitsreservoir von einer elastischen Wandung begrenzt ist, die sich unter Einwirkung des Gasdrucks elastisch verformt, so dass sich bei einer Verringerung des Gasdrucks das Volumen des Flüssigkeitsreservoirs verringert und dabei die Spülflüssigkeit aus dem Flüssigkeitsreservoir durch die Düse herausdrückt.

Durch die Elastizität der Flasche beziehungsweise der Wandungen kann auch dann noch Spülflüssigkeit ausgetragen werden, wenn der Gasdruck plötzlich nachlässt oder schwankt.

Des Weiteren kann erfindungsgemäß vorgesehen sein, dass das Druckgasreservoir eine Druckgaspatrone ist, bevorzugt eine Flüssiggaspatrone ist, besonders bevorzugt eine CO₂-Patrone ist, die lösbar mit der Druckgasleitung verbindbar oder verbunden ist, wobei die Druckgaspatrone bevorzugt über ein Öffnungsmittel für die Druckgaspatrone mit der Druckgasleitung verbindbar oder verbunden ist.

Die Anwendung einer Druckgaspatrone bewirkt, dass die Sprühvorrichtung unabhängig von einer externen Druckgasversorgung oder Stromversorgung ist. Alternativ könnte die Sprühvorrichtung auch mit einem Kompressor und einem Stromanschluss oder einem Akkumulator ausgestattet sein.

Bei Ausführungsformen mit Druckgaspatrone kann vorgesehen sein, dass in der Druckgasleitung zwischen dem Anschluss für die Druckgaspatrone und dem Überdruckventil ein Verdampfungsraum zur Verdampfung flüssiger Bestandteile eines verflüssigten Gases aus der Druckgaspatrone angeordnet ist, wobei das verdampfende verflüssigte Gas den Gasdruck erzeugt.

Hierdurch wird vermieden, dass flüssige Bestanteile des Gases aus der Druckgaspatrone oder direkt dahinter entstehender Schnee oder andere Kondensate tief in die Druckgasleitung vordringen und sich dort störend auswirken.
Ferner kann dabei vorgesehen sein, dass in der Druckgasleitung beim Anschluss für die Druckgaspatrone ein manuell betätigbares Ventil angeordnet ist.
Die Sprühvorrichtung kann dann einfach "scharf" geschaltet werden.
Alternativ zu einer Druckgaspatrone kann vorgesehen sein, dass das Druckgasreservoir an einen Kompressor angeschlossen ist, der bevorzugt über eine flexible Leitung mit der Sprühvorrichtung verbunden ist.
Der Kompressor kann auch Teil eines großflächigen Druckgasnetzes sein, das beispielsweise in einem Krankenhaus zur Verfügung steht.
Mit einer Weiterentwicklung der Erfindung wird vorgeschlagen, dass in der Druckgasleitung, bevorzugt zwischen dem Überdruckventil und dem Druckgasreservoir, ein Druckminderungsventil angeordnet ist, das den Gasdruck begrenzt, der in dem Flüssigkeitsreservoir auf die Spülflüssigkeit wirkt.
Mit dem Druckminderungsventil kann sichergestellt werden, dass in der folgenden, dahinter angeordneten Druckgasleitung und damit im Flüssigkeitsreservoir kein zu großer Druck entsteht, der zu einer Zerstörung von Teilen der Druckgasleitung oder der Wandungen des Flüssigkeitsreservoirs beziehungsweise der Flasche führen könnte. Zudem wird so erreicht, dass ein einigermaßen konstanter Druck auf die Spülflüssigkeit drückt und dadurch ein gleichmäßiger Spülflüssigkeitsstrom durch die Düse appliziert werden kann.
Dabei kann vorgesehen sein, dass zwischen dem Druckminderungsventil und dem Flüssigkeitsreservoir zumindest ein Sicherungselement angeordnet ist, insbesondere eine Berstscheibe und/oder ein zweites Überdruckventil angeordnet ist, das den Gasdruck begrenzt, der auf der Seite des Druckgasreservoirs auf dem Druckminderungsventil lastet.
Hierdurch kann sichergestellt werden, dass die Sprühvorrichtung auch bei einem Versagen des Überdruckventils oder des Druckminderungsventils noch sicher verwendet werden kann.
Mit einer besonders bevorzugten Ausführungsform der erfindungsgemäßen Sprühvorrichtung kann vorgesehen sein, dass die Düse mehrere Öffnungen hat, die in einem Winkel zueinander derart ausgerichtet sind, dass sich die aus den Öffnungen austretenden Spülflüssigkeitsstrahlen in einem Zerstäubungsraum der Düse treffen und dadurch den Sprühkegel aus der zerstäubten Spülflüssigkeit erzeugen.

Hierdurch wird auf einfachste Weise eine Vernebelung der Spülflüssigkeit erreicht, ohne dass hierfür ein Motor oder ein bewegliches Teil in der Düse notwendig wäre.

Bevorzugt treffen sich die Spülflüssigkeitsstrahlen in oder unmittelbar vor einer Austrittsöffnung der Düse.

Dabei kann vorgesehen sein, dass die Düse eine zentrale Öffnung zur Erzeugung eines mittigen Hauptstrahls und eine Mehrzahl von um die zentrale Öffnung herum angeordnete äußere Öffnungen aufweist, wobei bevorzugt bezogen auf die Hauptöffnung gegenüberliegende äußeren Öffnungen im gleichen Winkel in Richtung des Hauptstrahls geneigt sind.

Mit dieser Ausführung wird eine gute Vernebelung der Spülflüssigkeit erreicht und gleichzeitig ein kräftiger Sprühstrahl erzeugt.

Ferner kann vorgesehen sein, dass am Flüssigkeitseingang der Düse mindestens zwei Eingangsöffnungen angeordnet sind, so dass die in den Innenraum der Düse eintretende Spülflüssigkeit in mindestens zwei Spülflüssigkeitsströme aufgeteilt ist, die in der Düse derart zu wenigstens zwei Öffnungen geleitet werden, dass sich die mindestens zwei Spülflüssigkeitsstrahlen sich in einem Winkel von mindestens 10° vor der Austrittsöffnung der Düse treffen, bevorzugt sich die Spülflüssigkeitsstrahlen in einem Winkel zwischen 10° und 85° treffen, besonders bevorzugt in einem Winkel zwischen 15° und 45°.

Dadurch übernimmt die Düse alle für das Erzeugen des Sprühkegels wichtigen Funktionen, ohne dass der Aufbau dadurch komplex würde. Die Düse lässt sich einfach aus Kunststoff fertigen.

Es kann erfindungsgemäß auch vorgesehen sein, dass die Öffnungen der Düse in einem Winkel zueinander derart ausgerichtet sind, dass sich die aus den Öffnungen austretenden Spülflüssigkeitsstrahlen in einem Zerstäubungsraum und/oder einer Austrittsöffnung der Düse treffen.

Durch die Vernebelung beziehungsweise Zerstäubung in dem Zerstäubungsraum wird verhindert, dass sich unzerstäubte Flüssigkeitströpfchen von der Spitze der Düse lösen und unkontrolliert abtropfen. Zudem wird so ein gleichmäßigerer Sprühkegel erreicht.

Mit einer Weiterentwicklung der Erfindung wird auch vorgeschlagen, dass oberhalb der Spülflüssigkeit in dem Flüssigkeitsreservoir ein Gas enthalten ist, über das über die Oberfläche der Spülflüssigkeit ein Druck auf die Spülflüssigkeit ausübbar ist.

Mit einer Weiterentwicklung der Erfindung wird vorgeschlagen, dass die Düse als Spitze eines Austragsrohrs vorgesehen ist, wobei bevorzugt das Austragsrohr teleskopartig in axialer Richtung des Austragsrohrs verschiebbar zur Sprühvorrichtung angeordnet ist und/oder das Austragsrohr axial um einen Winkel von mindestens 30° drehbar gelagert ist.

Hierdurch kann die Sprühvorrichtung gut an unterschiedliche Gegebenheiten und Operationssituationen angepasst werden.

Die Aufgaben der Erfindung werden auch gelöst durch die Verwendung einer solchen medizinischen Sprühvorrichtung zum Erzeugen eines Sprühkegels zum Debridement von infiziertem Gewebe.

Ferner werden die Aufgaben der Erfindung auch gelöst durch ein Verfahren zum Erzeugen eines Sprühkegels einer medizinischen Spülflüssigkeit, insbesondere mit einer solchen Sprühvorrichtung, bei dem ein Gasdruck aus einem Druckgasreservoir durch eine Druckgasleitung in ein Flüssigkeitsreservoir der medizinischen Spülflüssigkeit geleitet wird und mit dem Gasdruck die Spülflüssigkeit aus dem Flüssigkeitsreservoir durch eine Düse gedrückt wird, wobei der Sprühkegel erzeugt wird, indem die Spülflüssigkeit durch die Düse strömt, wobei bei Übersteigen eines Grenzdrucks in der Druckgasleitung zumindest ein Überdruckventil in der Druckgasleitung geöffnet wird und dadurch das Druckgas in die Umgebung strömt und der Druck in der Druckgasleitung reduziert wird, bevorzugt begrenzt wird.

Durch diesen Sicherheitsmechanismus wird es gefahrlos möglich, einen Gasdruck direkt zum Erzeugen des Sprühkegels einer medizinischen Sprühvorrichtung zu verwenden.

Dabei kann vorgesehen sein, dass der Gasdruck durch Verdampfen eines Gases aus einer Flüssiggaspatrone, insbesondere einer CO₂-Patrone erzeugt wird, wobei bevorzugt das Gas teilweise in einem Verdampfungsraum verflüssigt wird, bevor es in Richtung des Überdruckventils geleitet wird.

Ferner kann vorgesehen sein, dass der Gasdruck mit einem Druckminderungsventil in der Druckgasleitung begrenzt wird und der durch das Druckminderungsventil begrenzte Gasdruck durch die Druckgasleitung zu dem Flüssigkeitsreservoir der medizinischen Spülflüssigkeit geleitet wird, wobei bevorzugt der Gasdruck zwischen dem Druckminderungsventil und dem Flüssigkeitsreservoir bei Übersteigen des Grenzdrucks reduziert wird, bevorzugt begrenzt wird.

Des Weiteren kann erfindungsgemäß vorgesehen sein, dass die Spülflüssigkeit durch mehrere Öffnungen einer Düse gedrückt wird und die so erzeugten Spülflüssigkeitsstrahlen mit einer derartigen Strömungsgeschwindigkeit und in einem derartigen Winkel aufeinander geschossen werden, dass die Spülflüssigkeitsstrahlen vor der Düse zerstäuben und einen Sprühkegel bilden.

Der Erfindung liegt die überraschende Erkenntnis zugrunde, dass es mit Hilfe eines Überdruckventils gelingt, einen Gasdruck bereitzustellen, der nach dem Öffnen des Überdruckventils auf die medizinische Spülflüssigkeit wirkt und dadurch diese durch eine Düse presst, wo die Spülflüssigkeit zu einem Sprühkegel vernebelt wird, ohne dass der Gasdruck so groß werden kann, dass der Behälter des Flüssigkeitsreservoirs des medizinischen Spülmittels durch einen zu großen Druck platzen könnte. Erst hierdurch wird überraschend, auf einfache Weise und in Abkehr vom Aufbau aller bisherigen Sprühvorrichtungen ein Aufbau ohne Motor möglich und eine direkte Verwendung des Druckgases möglich. Durch die direkte Verwendung des Gasdrucks als Antrieb für die Spülflüssigkeit benötigt das erfindungsgemäße Lavage-System keine Motoren und, keine rotierenden oder oszillierenden Teile zum Antrieb der Spülflüssigkeit. Hierdurch wird der Aufbau vereinfacht und somit die Fertigung des Lavage-Systems als nur einmal zu nutzender Wegwerfartikel möglich. Die Fertigung als Einmalartikel hat im medizinischen Bereich den Vorteil, dass keine Desinfektion des Lavage-Systems notwendig ist, bei der Fehler auftreten können und die bei einer Verwendung eines verunreinigten Lavage-Systems zu einer komplizierten Infektion beim Patienten führen können. Zudem kann der gesamte Aufbau sehr kostengünstig gestaltet werden.

Die Richtungsangaben davor oder dahinter beziehen sich auf die Flussrichtung des komprimierten Gases beziehungsweise der medizinischen Spülflüssigkeit.

Die Erfindung beruht auf der Idee, dass in einem Spülflüssigkeitsbehälter über der Spülflüssigkeit eine Gasphase übersteht und in diese ein ungiftiges Druckgas eingeleitet wird, so dass die überstehende Gasphase unter einem Überdruck gegenüber der Atmosphäre steht. Dadurch ist es möglich, die Spülflüssigkeit aus dem Spülflüssigkeitsbehälter zu pressen.

Die Idee besteht des Weiteren darin, aus einer kleinen Druckgaspatrone oder einem anderen Druckgasreservoir Druckgas in die der Spülflüssigkeit überstehende Gasphase einzuleiten, bis ein zuvor bestimmter Überdruck gegenüber dem umgebenden Atmosphärendruck erreicht wird. Danach öffnet mindestens ein Überdruckventil, so dass aus der Gaspatrone austretendes überschüssiges Druckgas in die Umgebung abgeblasen wird. Es verbleibt jedoch die unter Druck stehende Gasphase oberhalb der Spülflüssigkeit. Die unter Druck stehende Gasphase oberhalb der Spülflüssigkeit hat das Bestreben sich auszudehnen bis der Druckausgleich zur umgebenden Atmosphäre hergestellt ist. Dadurch wird die Spülflüssigkeit aus dem Spülflüssigkeitsbehälter ausgepresst. Die der Spülflüssigkeit überstehende Gasphase wirkt als elastische Gasfeder beziehungsweise als temporärer Energiespeicher. Wenn der Behälter des Flüssigkeitsreservoirs elastische Wände hat, wirken auch diese als Feder, so dass auch beim Öffnen des Überdruckventils noch weiter Spülflüssigkeit durch die Düse versprüht werden kann.

Es zeigte sich überraschend, dass bereits bei Überdrucken ab 0,4 bar ein kraftvolles Auspressen der Spülflüssigkeit möglich ist. Es wurde weiterhin überraschend gefunden, dass ab einem Überdruck von 0,4 bar bei Verwendung einer Sprühdüse, die den Sprühflüssigkeitsstrom im Inneren der Düse oder vor der Düse in mindestens zwei Ströme teilt, wobei diese im Inneren der Düse mit einem Winkel von mindestens 10° ineinander gelenkt werden, ein kraftvoller Sprühkegel entsteht, der aus Zufalls-verteilten Sprühflüssigkeitströpfchen besteht.

Durch Verwendung eines zusätzlichen Druckminderungsventils kann der geringe Überdruck in der überstehenden Gasphase bei Vergrößerung des Volumens der Gasphase infolge des Auspressens der Spülflüssigkeit konstant gehalten werden.

Ein besonders bevorzugtes Ausführungsbeispiel der Erfindung kann dabei wie folgt realisiert werden:
Das erfindungsgemäße medizinische Spülsystem ist dabei zusammengesetzt aus
a) einem gasdurchlässigen ersten Kanal,
b) mindestens einer Druckgaspatrone, die mit einem Öffnungsmittel mit der Druckgaspatrone lösbar, gasdurchlässig verbunden werden kann,
c) mindestens einem Verdampfungsbehälter beziehungsweise Verdampfungsraum, der über ein gasdurchlässiges Verbindungsmittel mit dem gasdurchlässigen ersten Kanal verbunden ist,
d) einem zweiten gasdurchlässigen zweiten Kanal, der gasundurchlässig vom gasdurchlässigen ersten Kanal getrennt ist, der über ein Verbindungsmittel mit dem Verdampfungsbehälter gasdurchlässig verbunden ist,
e) mindestens einem Überdruckventil, das mit dem gasdurchlässigen dritten Kanal gasdurchlässig verbunden ist, wobei bei Überdruck des Gases im gasdurchlässigen dritten Kanal das unter Überdruck stehende Gas in die umgebende Atmosphäre abgegeben werden kann,
f) mindestens einem Spülflüssigkeitsbehälter, der Spülflüssigkeit enthält, wobei eine Gasphase über der Spülflüssigkeit steht,
g) einem flexiblen gasdurchlässigen Verbindungsmittel, das mit dem gasdurchlässigen dritten Kanal verbunden ist, und das mit einem Spülflüssigkeitsvorratsbehälter gasdurchlässig verbunden ist,
h) einem flexiblen, flüssigkeitsdurchlässigen Verbindungsmittel, das mit dem Spülflüssigkeitsvorratsbehälter flüssigkeitsdurchlässig verbunden ist und das mit einem Austragsrohr und einer daran angeordneten Düse flüssigkeitsdurchlässig verbunden ist, und
i) mindestens ein Ventilelement, das den Spülflüssigkeitsstrom zwischen dem Spülflüssigkeitsbehälter und der am Austragsrohr angeordneten Düse reguliert.

Die Druckgaspatrone dient bei dieser Ausführung als Druckgasreservoir, der Spülflüssigkeitsbehälter als Flüssigkeitsreservoir, die Kanäle als Druckgasleitung und die Verbindungsmittel als Druckgasleitung beziehungsweise als Flüssigkeitsleitung.

Das Ventilelement ist bevorzugt mit einem manuell zu betätigenden Abzug verbunden, der durch mindestens eine Feder im nichtbetätigten Zustand so gehalten wird, dass der Spülflüssigkeitsstrom zwischen dem Spülflüssigkeitsbehälter und der am Austragsrohr angeordneten Düse durch das Ventilelement unterbrochen ist.

Dabei ist es für die medizinische Sprühvorrichtung wesentlich, dass die Gaspatrone ein nicht toxisches Gas enthält, beziehungsweise als Gas für das Gasreservoir ein nicht toxisches Gas verwendet wird. Als Gase, insbesondere für Gaspatronen, kommen Argon, Helium, Distickstoffmonoxid und Kohlendioxid in Betracht. Besonders ist bevorzugt, dass die Gaspatrone flüssiges Kohlendioxid enthält und dass Kohlendioxid als Druckgas bevorzugt wird. Kohlendioxid ist preisgünstig, nicht toxisch und hat den wesentlichen Vorteil, dass es bei Raumtemperatur in verflüssigter Form problemlos in Druckgaspatronen gelagert werden kann. Dadurch ist es möglich, große Gasvolumina in kleinvolumigen Druckgaspatronen bereit zu stellen.

Erfindungsgemäß kann auch vorgesehen sein, dass die über der Spülflüssigkeit stehende Gasphase einen Überdruck von 1 bis 10 bar gegenüber der umgebenden Atmosphäre hat, nachdem die Verbindung zum Gasreservoir herstellt ist, beziehungsweise nachdem die Gaspatrone geöffnet ist und das Druckgas in die überstehende Gasphase geströmt ist.

Erfindungsgemäß kann vorgesehen sein, dass das Verhältnis des Volumens der Gasphase, welche über der Spülflüssigkeit steht, zum Volumen der Spülflüssigkeit mindestens 1 zu 10 bis maximal 1 zu 1 beträgt.

Die Gaspatrone, die gasdurchlässigen Kanäle (erster, zweiter und dritter Kanal), der Verdampfungsbehälter, das mindestens eine Überdruckventil, die gasdurchlässigen Verbindungsmittel und der Ansatz der Austragsrohrs sind erfindungsgemäß bevorzugt in einem Gehäuse angeordnet, wobei das Gehäuse besonders bevorzugt pistolenförmig ausgebildet ist. Dadurch kann der medizinische Anwender die Sprühvorrichtung leicht greifen und bedienen.

Eine weitere vorteilhafte Ausgestaltung der Erfindung besteht darin, dass das Austragsrohr in axialer Richtung verschiebbar im Gehäuse angeordnet ist, wobei das Austragsrohr um seine Längsachse um eine Winkel von mindestens 30° drehbar gelagert ist, mindestens einen Zapfen an seinem der Düse entgegengesetzten Rohrende besitzt. Bei einer Ausführung mit Austragsrohr kann erfindungsgemäß bevorzugt vorgesehen sein, dass der Zapfen in eine schlitzförmige Führung des Gehäuses greift und mindestens zwei Aussparungen senkrecht zur schlitzförmigen Führung als Rast für den Zapfen angeordnet sind. Das bedeutet, die Länge des Austragsrohrs kann je nach dem gewünschten Anwendungszweck durch einfaches Heraus- oder Hereinschieben des Austragsrohrs in das Gehäuse variiert werden. Dadurch ist es möglich, ohne Zusatzaustragsrohre, Gewebeareale zu reinigen, bei denen ein kurzes Austragsrohr notwendig ist, wie zum Beispiel bei der Implantation von Knietotalgelenkendoprothesen, und es ist auch nach Herausziehen des Austragsrohrs möglich, Gewebeareale zu reinigen, bei denen ein langes Austragsrohr erforderlich ist, wie zum Beispiel bei der Implantation von Hüftschäften. Durch Drehen des Austragsrohrs um seine Längsachse kann der Zapfen des Austragsrohrs in der gewünschten Position durch Einrasten in die senkrecht zur Führung angeordneten Aussparungen nach dem Prinzip eines Bajonettverschlusses verriegelt werden.

Für die Sprühfunktion der Sprühvorrichtung kann erfindungsgemäß bevorzugt vorgesehen sein, dass am Flüssigkeitseingang der Düse mindestens zwei Öffnungen angeordnet sind, so dass die in den Innenraum der Düse eintretende Spülflüssigkeit in mindestens zwei Spülflüssigkeitsströme aufgeteilt wird, die im Innenraum der Düse so geleitet werden, dass diese sich in einem Winkel von mindestens 10° vor der Austrittsöffnung der Düse treffen. Durch das ineinander strömen beziehungsweise aufeinander schießen der mindestens zwei Spülflüssigkeitsströme wird die Spülflüssigkeit in kleinste Flüssigkeitströpfchen zerstäubt, die sich statistisch verteilt im Sprühkegel bewegen. Dadurch gibt es keine Spülflüssigkeitsstrahlen sondern Zufalls-verteilte einzelne Tröpfchen, so dass das gesamte zu reinigende Gewebeareal mit einzelnen Spülflüssigkeitströpfchen bei entsprechender Einwirkungszeit des Sprühkegels getroffen wird. Dadurch ist eine Reinigungswirkung der Sprühvorrichtung sicher gewährleistet.

Erfindungsgemäß ist auch ein Verfahren zum Erzeugen eines Sprühstrahls mit der erfindungsgemäßen Sprühvorrichtung. Das Verfahren kann dadurch charakterisiert sein, dass eine Druckgaspatrone durch ein Öffnungsmittel geöffnet wird, wobei das in der Gaspatrone enthaltene Gas und/oder verflüssigte Gas in den gasdurchlässigen ersten Kanal strömt, von diesem über das gasdurchlässiges Verbindungsmittel in den Verdampfungsbehälter strömt, in dem Verdampfungsbehälter verdampft, das im Verdampfungsbehälter gebildete Gas durch ein gasdurchlässiges Verbindungsmittel in einen gasdurchlässigen zweiten Kanal, von dort über ein flexibles gasdurchlässiges Verbindungsmittel in den Spülflüssigkeitsbehälter, wobei sich dadurch in dem Spülflüssigkeitsbehälter ein Überdruck von 1 bis 10 bar bildet über dem der Spülflüssigkeit befindlichen Gasraum, wodurch die Spülflüssigkeit durch ein mit dem Spülflüssigkeitsbehälter verbundenes flexiblen Verbindungsmittel in ein Ausströmrohr gepresst wird und von dort in eine Düse gelangt und aus dieser Düse als Spülmittelstrahl in die Umgebung austritt.

Dabei kann vorgesehen sein, dass nach Erreichen des Öffnungsdrucks des mindestens einen Überdruckventils im Bereich von 1 bis 10 bar überschüssiges Druckgas über ein zusätzliches Überdruckventil an die Umgebung abgegeben wird.

Im Folgenden werden Ausführungsbeispiele der Erfindung anhand von zwei schematisch dargestellten Figuren erläutert, ohne jedoch dabei die Erfindung zu beschränken. Dabei zeigt:
Figur 1: eine schematische Querschnittansicht durch eine erfindungsgemäße medizinische Sprühvorrichtung; und
Figur 2: eine schematische perspektivische Ansicht auf eine Düse und ein teleskopartiges Austragsrohr einer erfindungsgemäßen medizinischen Sprühvorrichtung.

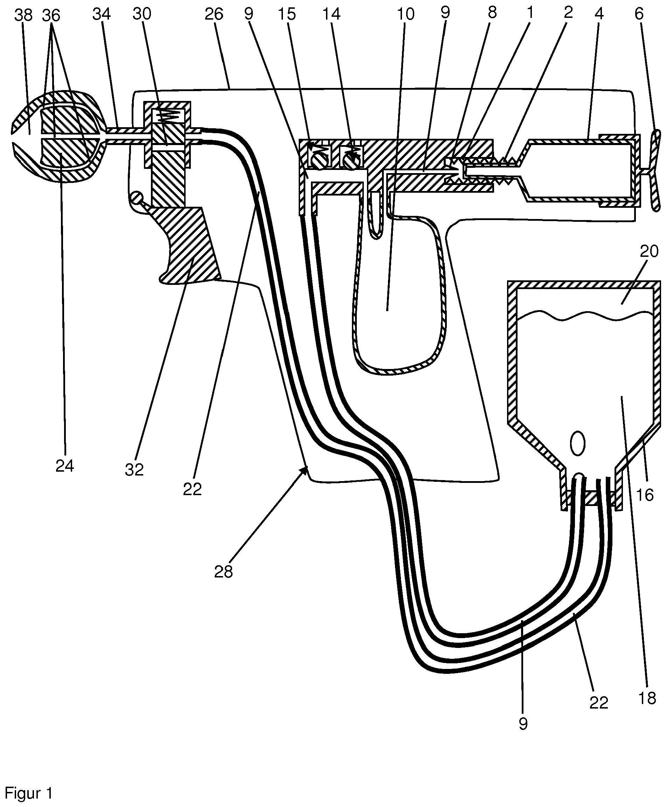

Figur 1 zeigt eine schematische Querschnittansicht durch eine erfindungsgemäße medizinische Sprühvorrichtung. An der Rückseite der Sprühvorrichtung ist eine Halterung mit einem Innengewinde 1 zur Aufnahme eines Außengewindes 2 einer CO₂-Druckgaspatrone 4 vorgesehen. Am Boden der Druckgaspatrone 4 ist ein Drehgriffstück 6 befestigt, um das Eindrehen und Befestigen der Druckgaspatrone 4 in die Halterung der Sprühvorrichtung zu erleichtern.

In der Halterung ist ein Hohldorn 8 angeordnet, der zum Öffnen der Druckgaspatrone 4 dient und der mit einer Druckgasleitung 9 verbunden ist. Beim Eindrehen der Druckgaspatrone 4 wird diese mit einem zum Öffnen vorgesehenen Verschluss auf den Hohldorn 8 aufgedrückt, so dass sich die Druckgaspatrone 4 öffnet und das Druckgas auf der Druckgaspatrone 4 in den Hohldorn 8 und damit in die Druckgasleitung 9 strömt. In der Druckgasleitung 9 ist ein Verdampfungsraum 10 beziehungsweise ein Verdampfungsbehälter 10 angeordnet. Flüssige Bestandteile des CO₂-Gases oder andere schneeartige Kondensate, die aus der Druckgaspatrone in die Druckgasleitung 9 gelangen, werden dort aufgefangen und können dort allmählich verdampfen. Durch diesen Aufbau wird vermieden, dass flüssige oder schneeartige Bestandteile tiefer in die Druckgasleitung 9 vordringen und dort beim Verdampfen zu Unregelmäßigkeiten des Drucks führen.

Alternativ zur Verwendung einer Druckgaspatrone 4 kann auch vorgesehen sein, dass an die Druckgasleitung 9 ein Verbindungsschlauch (nicht gezeigt) einer Druckgasquelle wie beispielsweise einem Kompressor und/oder einer zentralen Druckgasverteilung (nicht gezeigt) angeschlossen ist. Dann kann unter normalen Umständen auch auf den Verdampfungsraum 10 verzichtet werden.

Im weiteren Verlauf der Druckgasleitung 9 sind zwei Überdruckventile 14, 15 angeordnet, die ab einem Grenzdruck zwischen 1 und 10 bar die Druckgasleitung 9 nach außen in Richtung der Umgebung der Sprühvorrichtung öffnen. Die Überdruckventile 14, 15 sind beispielsweise durch mit Stahlfedern gelagerte Kugeln in einem zylindrischen Hohlraum aufgebaut, wobei die Kugeln durch die Stahlfedern auf einer Kugeloberfläche in Richtung der Druckgasleitung 9 gedrückt werden und dadurch die Druckgasleitung 9 abdichten. Der zylindrische Hohlraum weist zumindest eine Verbindung nach außen an die Umgebung der Sprühvorrichtung auf, die nicht durch die Kugel verdeckt werden kann.

Zwischen den Überdruckventilen 14, 15 und dem Verdampfungsraum 10 kann ein Druckminderungsventil (nicht gezeigt) vorgesehen sein, mit dem der Druck in der weiteren Druckgasleitung 9 auf einen passenden maximalen Gasdruck begrenzt wird. Wie dies bei Druckminderungsventile häufig der Fall ist, kann auch bei einem hier verwendeten Druckminderungsventil vorgesehen sein, dass der durch das Druckminderungsventil eingestellte Druck durch eine Stellschraube (nicht gezeigt) eingestellt und manuell verändert werden kann.

Hinter den als blockierbare T-Stücke ausgeformten Überdruckventilen 14, 15 setzt sich die Druckgasleitung 9 als flexibler Schlauch fort, der einen oder mehrere Meter aus der Sprühvorrichtung herausführt und dort über einen Stopfen oder ein anderes Verbindungsmittel mit einer kopfüber aufgehängten flexiblen Flasche 16 aus einem elastischen Kunststoff verbunden ist. In der Flasche 16 ist eine medizinische Spülflüssigkeit 18 zur Behandlung einer Wunde und eine überstehende Gasphase 20 enthalten.

Der Überdruck aus der Druckgasleitung 9 mündet in die elastische Flasche 16 ein und dehnt die überstehende Gasphase 20 und die Flasche 16 aus. Durch den Gasdruck aus der Druckgasleitung 9 und durch den elastischen Druck der Flasche 16 steht die medizinische Spülflüssigkeit 18 unter Druck und wird durch eine Flüssigkeitsleitung 22 in Richtung einer Düse 24 der Spülvorrichtung gedrückt. Die Flüssigkeitsleitung 22 ist vorliegend ein flexibler Schlauch, der durch den gleichen Stopfen wie der flexible Schlauch der Druckgasleitung 9 in die Flasche 16 eingeführt ist. Der Stopfen dichtet die Flasche 16 ab.

Die meisten Bestandteile der medizinischen Sprühvorrichtung sind in einem Gehäuse 26 aus Plastik angeordnet, das fest mit den restlichen Teilen verbunden ist und das die Form einer Pistole mit einem Pistolengriff 28 hat. Die Flüssigkeitsleitung 22 und der flexible Teile der Druckgasleitung 9, die außerhalb des Gehäuses 26 angeordnet sind, können in einem gemeinsamen, flexiblen Schlauch (nicht gezeigt) eingeschlossen sein, um ein Verheddern der Flüssigkeitsleitung 22 und der externen Druckgasleitung 9 zu verhindern.

Zwischen der Düse 24 und der Flüssigkeitsleitung 22 ist im Inneren des Gehäuses 26 ein manuell bedienbares, mit einer Stahlfeder gefedertes Ventilelement 30 angeordnet, das mit einem drehbar gelagerten Abzug 32 bedienbar ist. In Figur 1 ist das Ventilelement 30 in der geschlossenen Position gezeigt. Hinter dem Ventilelement 30 wird die Flüssigkeitsleitung 22 durch ein Austragsrohr 34 zur Düse 24 geleitet. Es ist bevorzugt, dass das Austragsrohr 34 teleskopartig ausfahrbar ist (nicht gezeigt). Ferner kann die Düse 24 gegen die Achse des Austragsrohrs 34 geneigt und drehbar gelagert sein.

Wenn das Ventilelement 30 über den Abzug 32 bedient wird, wird eine durchgehende Leitung der Spülflüssigkeit 18 aus der Flasche 16 bis in die Düse 24 gebildet. In der Düse 24 sind mehrere Kanäle 36 vorgesehen, so dass der Flüssigkeitsstrom der Spülflüssigkeit 18 innerhalb der Düse 24 in mehrere Flüssigkeitsströme geteilt wird. Die Kanäle 36 sind so geführt, dass die hinter der Düse 24 ausströmenden Spülflüssigkeitsstrahlen (nicht gezeigt) in einem Winkel zwischen 10° und 80° in einem Zerstäubungsraum 38 der in einer Austrittsöffnung der Düse 24 aufeinander treffen, beziehungsweise aufeinander geschossen werden. Die äußeren Spülflüssigkeitsstrahlen können dabei entlang der Innenwandung des Zerstäubungsraums 38 laufen und treffen im Bereich der zentralen Austrittsöffnung (in Figur 1 links) der Düse 24 auf den zentralen Hauptstrahl. Die aufeinandertreffenden Spülflüssigkeitsstrahlen zerstäuben beziehungsweise vernebeln dabei aufgrund ihrer kinetischen Energie im Zerstäubungsraum 38 zu einem Sprühkegel feiner Spülflüssigkeitströpfchen (nicht gezeigt), der durch die vordere Austrittsöffnung austritt.

Mit der Vorrichtung kann so auf einfachste Weise ein Sprühkegel einer medizinischen Spülflüssigkeit erzeugt werden, ohne dass hierfür ein Motor oder andere sich ständig bewegende Teile benötigt würden. Der Aufbau kann im Wesentlichen aus Kunststoffteilen aufgebaut werden, die durch einfache Spitzgussverfahren herstellbar sind.

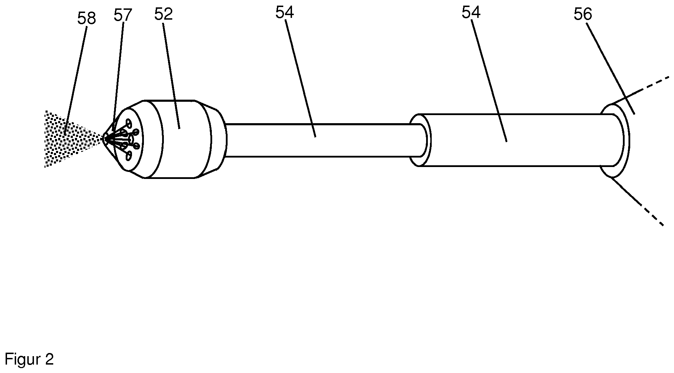

Figur 2 zeigt eine schematische perspektivische Ansicht auf eine Düse 52 und ein teleskopartiges Austragsrohr 54 einer erfindungsgemäßen medizinischen Sprühvorrichtung. Das Austragsrohr 54 ragt aus einem Gehäuse 56 der Sprühvorrichtung. Die restliche Sprühvorrichtung entspricht beispielsweise dem Aufbau nach Figur 1. Die Düse 52 ist außen rotationssymmetrisch geformt.

Im Inneren der Düse 52 wird ein Flüssigkeitsstrom einer durch das Austragsrohr 54 fließenden medizinischen Spülflüssigkeit in sechs Teilströme aufgeteilt, die durch sechs Öffnungen auf der in Figur 2 zu sehenden Vorderfläche der Düse 52 münden. Die Vorderfläche der Düse 52 ist in Richtung des Mittelpunkts der Düse 52 gewölbt. Vor dieser Vorderfläche ist eine konische Kappe aus einem transparenten Kunststoff angeordnet. Der Kunststoff muss nicht transparent sein, dies erleichtert vorliegend aber die Beschreibung der Düsenfunktion mit der Figur 2. Zwischen der konischen Kappe und der Vorderfläche der Düse 52 bildet sich ein Zerstäubungsraum 57. Der Zerstäubungsraum 57 hat eine zentrale Austrittsöffnung, in der sich Flüssigkeitsstrahlen aus den Öffnungen treffen.

Die sieben Flüssigkeitsleitungen im Inneren der Düse 52, sind bis auf die für den mittleren Hauptstrahl im Bereich der Öffnungen in Richtung der Symmetrieachse der äußeren Form der Düse 52 geneigt. Die Neigungen der Leitungen haben gegenüber der Symmetrieachse der äußeren Form der Düse 52 beziehungsweise gegenüber dem zentralen Hauptstrahl alle den gleichen Winkel und die sechs Öffnungen sind symmetrisch um diese Symmetrieachse beziehungsweise den Hauptstrahl herum in gleichen Abständen davon auf der Vorderfläche der Düse 52 verteilt.

Dadurch treffen sieben Spülflüssigkeitsstrahlen (in Figur 2 durch Striche angedeutet), die aus den Öffnungen kommen, alle in einem Bereich (der Austrittsöffnung) aufeinander, zerstäuben beziehungsweise vernebeln in dem Zerstäubungsraum 57 und bilden einen Sprühkegel 58 der medizinischen Flüssigkeit vor der Düse 52, wenn eine Spülflüssigkeit von der Rückseite der Düse 52 aus von der Sprühvorrichtung in die Düse 52 gedrückt wird.

Der teleskopartige Aufbau des Austragsrohrs 54 dient dazu, die Sprühvorrichtung bei unterschiedlichen Einsatzorten universell einsetzbar zu machen. Dazu kann erfindungsgemäß bevorzugt die Neigung der Düse 52 gegenüber dem Austragsrohr 54 einstellbar sein.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Innengewinde
- 2: Außengewinde
- 4: Druckgasreservoir/Druckgaspatrone
- 6: Drehgriffstück
- 8: Hohldorn
- 9: Druckgasleitung
- 10: Verdampfungsraum
- 14: Überdruckventil
- 15: Überdruckventil
- 16: Flasche
- 18: Medizinische Spülflüssigkeit
- 20: Überstehende Gasphase
- 22: Flüssigkeitsleitung
- 24: Düse
- 26: Gehäuse
- 28: Pistolengriff
- 30: Ventilelement
- 32: Abzug
- 34: Austragsrohr
- 36: Kanal
- 38: Zerstäubungsraum
- 52: Düse
- 54: Austragsrohr
- 56: Gehäuse
- 57: Zerstäubungsraum
- 58: Sprühkegel

## Patentansprüche

1. Medizinische Sprühvorrichtung zum Ausspülen einer Wunde, insbesondere Lavage-System, aufweisend ein Flüssigkeitsreservoir (16) für eine medizinische Spülflüssigkeit (18) oder einen Anschluss für ein solches Flüssigkeitsreservoir (16) und ein Druckgasreservoir (4), wobei das Druckgasreservoir (4) über eine Druckgasleitung (9) mit dem Flüssigkeitsreservoir (16) verbunden oder verbindbar ist, so dass die Spülflüssigkeit (18) von dem auf die Spülflüssigkeit (18) wirkenden Gasdruck des Druckgasreservoirs (4) durch eine Düse (24, 52) drückbar ist, um einen Sprühkegel (58) zu erzeugen, **dadurch gekennzeichnet, dass** in der Wandung der Druckgasleitung (9) wenigstens ein Überdruckventil (14, 15) angeordnet ist, das die Druckgasleitung (9) in Abhängigkeit von dem Gasdruck aus dem Druckgasreservoir (4) nach außen öffnet und schließt.

2. Sprühvorrichtung nach Anspruch 1, **dadurch gekennzeichnet, dass** das Flüssigkeitsreservoir (16) über eine Flüssigkeitsleitung (22) mit der Düse (24, 52) verbunden oder verbindbar ist, wobei in der Flüssigkeitsleitung (22) ein manuell betätigbares Ventilelement (30) angeordnet ist, das bevorzugt zur Steuerung des Volumenstroms der Spülflüssigkeit (18) geeignet ist und das besonders bevorzugt mit einem Abzug (32) bedienbar ist.

3. Sprühvorrichtung nach Anspruch 1 oder 2, **dadurch gekennzeichnet, dass** das Flüssigkeitsreservoir (16) eine Flasche (16) mit einer medizinischen Spülflüssigkeit (18) ist, die über die Druckgasleitung (9) und/oder über die Flüssigkeitsleitung (22) mit der Sprühvorrichtung verbunden oder verbindbar ist, wobei bevorzugt die Flüssigkeitsleitung (22) und die Druckgasleitung (9) durch die gleiche Öffnung der im Betrieb kopfüber angeordneten Flasche (16) münden.

4. Sprühvorrichtung nach einem der vorangehenden Ansprüche, **dadurch gekennzeichnet, dass**
das Flüssigkeitsreservoir (16) von einer elastischen Wandung begrenzt ist, die sich unter Einwirkung des Gasdrucks elastisch verformt, so dass sich bei einer Verringerung des Gasdrucks das Volumen des Flüssigkeitsreservoirs (16) verringert und dabei die Spülflüssigkeit (18) aus dem Flüssigkeitsreservoir (16) durch die Düse (24, 52) herausdrückt.

5. Sprühvorrichtung nach einem der vorangehenden Ansprüche, **dadurch gekennzeichnet, dass**
das Druckgasreservoir (4) eine Druckgaspatrone (4) ist, bevorzugt eine Flüssiggaspatrone ist, besonders bevorzugt eine CO₂-Patrone ist, die lösbar der Druckgasleitung (9) verbindbar oder verbunden ist, wobei die Druckgaspatrone (4) bevorzugt über ein Öffnungsmittel (8) für die Druckgaspatrone (4) mit der Druckgasleitung (9) verbindbar oder verbunden ist.

6. Sprühvorrichtung nach Anspruch 5, **dadurch gekennzeichnet, dass** in der Druckgasleitung (9) zwischen dem Anschluss für die Druckgaspatrone (4) und dem Überdruckventil (14, 15) ein Verdampfungsraum (10) zur Verdampfung flüssiger Bestandteile eines verflüssigten Gases aus der Druckgaspatrone angeordnet ist, wobei das verdampfende verflüssigte Gas den Gasdruck erzeugt.

7. Sprühvorrichtung nach Anspruch 5 oder 6, **dadurch gekennzeichnet, dass** in der Druckgasleitung (9) beim Anschluss für die Druckgaspatrone (4) ein manuell betätigbares Ventil angeordnet ist.

8. Sprühvorrichtung nach einem der vorangehenden Ansprüche, **dadurch gekennzeichnet, dass**
in der Druckgasleitung (9), bevorzugt zwischen dem Überdruckventil (14, 15) und dem Druckgasreservoir (4), ein Druckminderungsventil angeordnet ist, das den Gasdruck begrenzt, der in dem Flüssigkeitsreservoir (16) auf die Spülflüssigkeit (18) wirkt.

9. Sprühvorrichtung nach Anspruch 8, **dadurch gekennzeichnet, dass** zwischen dem Druckminderungsventil und dem Flüssigkeitsreservoir (16) zumindest ein Sicherungselement angeordnet ist, insbesondere eine Berstscheibe und/oder ein zweites Überdruckventil angeordnet ist, das den Gasdruck begrenzt, der auf der Seite des Druckgasreservoirs (4) auf dem Druckminderungsventil lastet.

10. Sprühvorrichtung nach einem der vorangehenden Ansprüche, **dadurch gekennzeichnet, dass**
die Düse (24, 52) mehrere Öffnungen hat, die in einem Winkel zueinander derart ausgerichtet sind, dass sich die aus den Öffnungen austretenden Spülflüssigkeitsstrahlen in einem Zerstäubungsraum (38, 57) der Düse (24, 52) treffen und dadurch den Sprühkegel (58) aus der zerstäubten Spülflüssigkeit (18) erzeugen.

11. Sprühvorrichtung nach Anspruch 10, **dadurch gekennzeichnet, dass**
die Düse (24, 52) eine zentrale Öffnung zur Erzeugung eines mittigen Hauptstrahls und eine Mehrzahl von um die zentrale Öffnung herum angeordnete äußere Öffnungen aufweist, wobei bevorzugt bezogen auf die Hauptöffnung gegenüberliegende äußeren Öffnungen im gleichen Winkel in Richtung des Hauptstrahls geneigt sind.

12. Sprühvorrichtung nach einem der vorangehenden Ansprüche, **dadurch gekennzeichnet, dass**
oberhalb der Spülflüssigkeit (18) in dem Flüssigkeitsreservoir (16) ein Gas (20) enthalten ist, über das über die Oberfläche der Spülflüssigkeit (18) ein Druck auf die Spülflüssigkeit (18) ausübbar ist.

13. Verfahren zum Erzeugen eines Sprühkegels (58) einer medizinischen Spülflüssigkeit (18), insbesondere mit einer Sprühvorrichtung nach einem der Ansprüche 1 bis 12, bei dem ein Gasdruck aus einem Druckgasreservoir (4) durch eine Druckgasleitung (9) in ein Flüssigkeitsreservoir (16) der medizinischen Spülflüssigkeit (18) geleitet wird und mit dem Gasdruck die Spülflüssigkeit (18) aus dem Flüssigkeitsreservoir (16) durch eine Düse (24, 52) gedrückt wird, wobei der Sprühkegel (58) erzeugt wird, indem die Spülflüssigkeit (18) durch die Düse (24, 52) strömt, **dadurch gekennzeichnet, dass** bei Übersteigen eines Grenzdrucks in der Druckgasleitung (9) zumindest ein Überdruckventil (14, 15) in der Druckgasleitung (9) geöffnet wird und dadurch das Druckgas in die Umgebung strömt und der Druck in der Druckgasleitung (9) reduziert wird, bevorzugt begrenzt wird.

14. Verfahren nach Anspruch 13, **dadurch gekennzeichnet, dass**
der Gasdruck durch Verdampfen eines Gases aus einer Flüssiggaspatrone (4), insbesondere einer CO₂-Patrone (4) erzeugt wird, wobei bevorzugt das Gas teilweise in einem Verdampfungsraum (10) verflüssigt wird, bevor es in Richtung des Überdruckventils (14, 15) geleitet wird.

15. Verfahren nach Anspruch 13 oder 14, **dadurch gekennzeichnet, dass**
der Gasdruck mit einem Druckminderungsventil in der Druckgasleitung (9) begrenzt wird und der durch das Druckminderungsventil begrenzte Gasdruck durch die Druckgasleitung (9) zu dem Flüssigkeitsreservoir (16) der medizinischen Spülflüssigkeit (18) geleitet wird, wobei bevorzugt der Gasdruck zwischen dem Druckminderungsventil und dem Flüssigkeitsreservoir (16) bei Übersteigen des Grenzdrucks reduziert wird, bevorzugt begrenzt wird.

## Claims

1. A medical spraying device for irrigating a wound, in particular lavage system, comprising a liquid reservoir (16) for a medical irrigation liquid (18) or a connection for such a liquid reservoir (16) and a compressed gas reservoir (4), wherein the compressed gas reservoir (4) is connected or connectable via a pressure line (9) to the liquid reservoir (16), such that the irrigation liquid (18) can be pushed by the gas pressure of the compressed gas reservoir (4) acting on the irrigation liquid (18) through a nozzle (24, 52) in order to produce a spray cone (58), **characterized in that**
at least one pressure relief valve (14, 15) is arranged in the wall of the pressure line (9) which opens outwardly and closes the pressure line (9) in accordance with the gas pressure from the compressed gas reservoir (4).

2. The spraying device according to Claim 1, **characterised in that**
the liquid reservoir (16) is connected or connectable via a liquid line (22) to the nozzle (24, 52), wherein a manually actuatable valve element (30) is arranged in the liquid line (22), is preferably suitable for controlling the volume flow rate of the irrigation liquid (18), and is particularly preferably operable using a trigger (32).

3. The spraying device according to Claim 1 or 2, **characterised in that**
the liquid reservoir (16) is a bottle (16) containing the medical irrigation liquid (18) which is connected or connectable via the pressure line (9) and/or via the liquid line (22) to the spraying device, wherein preferably the liquid line (22) and the pressure line (9) discharge through the same opening in the bottle (16) arranged head-down during operation.

4. The spraying device according to one of the preceding claims, **characterised in that**
the liquid reservoir (16) is delimited by a resilient wall, which deforms resiliently under the action of the gas pressure, such that, with a reduction of the gas pressure, the volume of the liquid reservoir (16) reduces and in so doing pushes the irrigation liquid (18) out from the liquid reservoir (16) through the nozzle (24, 52).

5. The spraying device according to one of the preceding claims, **characterised in that**
the compressed gas reservoir (4) is a compressed gas cartridge (4), preferably a liquefied gas cartridge, particularly preferably a CO₂ cartridge, which is detachably connectable or connected to the pressure line (9), wherein the compressed gas cartridge (4) is preferably connectable or connected to the pressure line (9) via an opening means (8) for the compressed gas cartridge (4).

6. The spraying device according to Claim 5, **characterised in that**
an evaporation space (10) for the evaporation of liquid constituents of a liquefied gas from the compressed gas cartridge is arranged in the pressure line (9) between the connection for the compressed gas cartridge (4) and the pressure relief valve (14, 15), wherein the evaporating liquefied gas produces the gas pressure.

7. The spraying device according to Claim 5 or 6, **characterised in that**
a manually actuatable valve is arranged in the pressure line (9) at the connection for the compressed gas cartridge (4).

8. The spraying device according to one of the preceding claims, **characterised in that**
a pressure reduction valve (12) is arranged in the pressure line (9), preferably between the pressure relief valve (14, 15) and the compressed gas reservoir (4), which limits the gas pressure acting in the liquid reservoir (16) on the irrigation liquid (18).

9. The spraying device according to Claim 8, **characterised in that**
at least one safety element is arranged between the pressure reduction valve and the liquid reservoir (16), in particular a bursting disc and/or a second pressure relief valve, which limits the gas pressure loading the pressure reduction valve on the side of the compressed gas reservoir (4).

10. The spraying device according to one of the preceding claims, **characterised in that**
the nozzle (24, 52) has a number of openings, which are arranged at an angle to one another in such a way that the irrigation liquid jets exiting from the openings meet in an atomisation space (38, 57) of the nozzle (24, 52) and thus produce the spray cone (58) from the atomised irrigation liquid (18).

11. The spraying device according to Claim 10, **characterised in that**
the nozzle (24, 52) has a central opening for producing a middle main jet and a plurality of outer openings arranged around the central opening, wherein preferably outer openings that are opposite one another with respect to the main opening are preferably inclined at the same angle in the direction of the main jet.

12. The spraying device according to one of the preceding claims, **characterised in that**
a gas (20) is contained above the irrigation liquid (18) in the liquid reservoir (16), via this gas (20) a pressure can be administered onto the irrigation liquid (18) via the surface of the irrigation liquid (18).

13. A method for producing a spray cone (58) of a medical irrigation liquid (18), in particular using a spraying device according to one of Claims 1 to 12, in which a gas pressure is conveyed from a compressed gas reservoir (4) through a pressure line (9) into a liquid reservoir (16) of the medical irrigation liquid (18), and the irrigation liquid (18) is pushed out from the liquid reservoir (16) through a nozzle (24, 52) by means of the gas pressure, wherein the spray cone (58) is produced in that the irrigation liquid (18) flows through the nozzle (24, 52), **characterized in that**, if a limit pressure in the pressure line (9) is exceeded, at least one pressure relief valve (14, 15) in the pressure line (9) is opened and the compressed gas thus flows into the surrounding environment and the pressure in the pressure line (9) is reduced, preferably limited.

14. The method according to Claim 13, **characterised in that**
the gas pressure is produced by evaporating a gas from a liquid gas cartridge (4), in particular a CO₂ cartridge (4), wherein the gas is preferably liquefied in part in an evaporation space (10) before it is conveyed in the direction of the pressure relief valve (14, 15).

15. The method according to Claim 13 or 14, **characterised in that**
the gas pressure is limited using a pressure reduction valve in the pressure line (9), and the gas pressure limited by the pressure reduction valve is conveyed through the pressure line (9) to the liquid reservoir (16) of the medical irrigation liquid (18), wherein the gas pressure between the pressure reduction valve and the liquid reservoir (16) is preferably reduced, preferably limited, if the limit pressure is exceeded.

## Revendications

1. Dispositif de vaporisation médical pour le rinçage d'une plaie, en particulier, système de lavage, présentant un réservoir de liquide (16) destiné à un liquide de lavage (18) médical, ou une connexion pour un tel réservoir de liquide (16) et un réservoir de gaz sous pression (4), le réservoir de gaz sous pression (4) étant relié ou pouvant être relié avec le réservoir de liquide (16) par le biais d'une conduite de gaz sous pression (9) de sorte que le liquide de lavage (18) peut être réglé au niveau de la pression par la pression de gaz du réservoir de gaz sous pression (4) agissant sur le liquide de lavage (18) à travers une buse (24, 52), afin de générer un cône de vaporisation (58), **caractérisé en ce qu'**au moins une soupape de surpression (14, 15), qui s'ouvre vers l'extérieur et se ferme en fonction de la pression du gaz provenant du réservoir de gaz sous pression (4), est disposée dans la paroi de la conduite de gaz sous pression (9).

2. Dispositif de vaporisation selon la revendication 1, **caractérisé en ce que**
le réservoir de liquide (16) est relié ou peut être relié avec la buse (24, 52) par l'intermédiaire d'une conduite de liquide (22), où un élément de soupape (30), pouvant être actionné manuellement, qui est de préférence approprié pour la commande du flux volumique du liquide de lavage (18) et qui peut être de manière particulièrement préférée actionné avec un système d'aspiration (32), est disposé dans la conduite de liquide (22).

3. Dispositif de vaporisation selon la revendication 1 ou la revendication 2, **caractérisé en ce que**
le réservoir de liquide (16) est une bouteille (16) avec un liquide de lavage (18) médical qui est reliée ou peut être reliée avec le dispositif de vaporisation par l'intermédiaire de la conduite de gaz sous pression (9) et/ou par l'intermédiaire de la conduite de liquide (22), où, de préférence, la conduite de liquide (22) et la conduite de gaz sous pression (9) débouchent par le même orifice de la bouteille (16) disposée tête en bas lors du fonctionnement.

4. Dispositif de vaporisation selon l'une des revendications précédentes, **caractérisé en ce que**
le réservoir de liquide (16) est délimité par une paroi élastique qui se déforme élastiquement sous l'effet de la pression du gaz de sorte que le volume du réservoir de liquide (16) diminue lors d'une diminution de la pression de gaz et pousse ainsi le liquide de lavage (16) hors du réservoir de liquide (16) à travers la buse (24, 52).

5. Dispositif de vaporisation selon l'une des revendications précédentes, **caractérisé en ce que**
le réservoir de gaz sous pression (4) est une cartouche de gaz sous pression (4), de préférence, est une cartouche de gaz liquéfié, de manière particulièrement préférée, est une cartouche de CO₂, qui peut être reliée ou est reliée de manière amovible avec la conduite de gaz sous pression (9), la cartouche de gaz sous pression (4) pouvant être reliée ou étant reliée avec la conduite de gaz sous pression (9) de préférence par l'intermédiaire d'un système d'ouverture (8) pour la cartouche de gaz sous pression (4).

6. Dispositif de vaporisation selon la revendication 5, **caractérisé en ce que**
dans la conduite de gaz sous pression (8), une chambre d'évaporation (10) est disposée entre la connexion pour la cartouche de gaz sous pression (4) et la soupape de surpression (14, 15) pour l'évaporation de composants liquides d'un gaz liquéfié à partir de la cartouche de gaz sous pression, le gaz liquéfié évaporé générant la pression de gaz.

7. Dispositif de vaporisation selon la revendication 5 ou la revendication 6, **caractérisé en ce que**,
dans la conduite de gaz sous pression (9), une soupape pouvant être actionnée manuellement est disposée près du raccord pour la cartouche de gaz sous pression (4).

8. Dispositif de vaporisation selon l'une des revendications précédentes, **caractérisé en ce que**,
dans la conduite de gaz sous pression (9), une soupape de réduction de la pression, qui limite la pression du gaz qui agit sur le liquide de lavage (18) dans le réservoir de liquide (16), est disposée de préférence entre la soupape de surpression (14, 15) et le réservoir de gaz sous pression (4).

9. Dispositif de vaporisation selon la revendication 8, **caractérisé en ce qu'**au
moins un élément de sécurité est disposé entre la soupape de réduction de la pression et le réservoir de liquide (16), notamment un disque de rupture et/ou une deuxième soupape de surpression qui limite la pression du gaz qui agit sur le côté du réservoir de gaz sous pression (4) sur la soupape de réduction de la pression.

10. Dispositif de vaporisation selon l'une des revendications précédentes, **caractérisé en ce que**
la buse (24, 52) a plusieurs orifices qui sont orientés les uns par rapport aux autres avec un angle tel que les courants de liquide de lavage sortant des orifices se rassemblent dans une chambre de nébulisation (38, 57) de la buse (24, 52) et génèrent ainsi le cône de vaporisation (58) à partir du liquide de lavage (18) vaporisé.

11. Dispositif de vaporisation selon la revendication 10, **caractérisé en ce que**
la buse (24, 52) présente un orifice central pour la formation d'un flux principal central et une multiplicité d'orifices extérieurs disposés autour de l'orifice central, où, de préférence, les orifices extérieurs situés en face par rapport à l'orifice principal sont inclinés en direction du courant principal avec le même angle.

12. Dispositif de vaporisation selon l'une des revendications précédentes, **caractérisé en ce**
**qu'**un gaz (20) est contenu au-dessus du liquide de lavage (18) dans le réservoir de liquide (16) par l'intermédiaire duquel une pression peut être exercée sur le liquide de lavage (18) par le biais de la surface du liquide de lavage (18).

13. Procédé de génération d'un cône de vaporisation (58) d'un liquide de lavage (18) médical, notamment avec un dispositif de vaporisation selon l'une des revendications 1 à 12, chez lequel une pression de gaz est menée à partir d'un réservoir de gaz sous pression (4) dans un réservoir de liquide (16) du liquide de lavage (18) médical à travers une conduite de gaz sous pression (9) et, à l'aide de la pression du gaz, le liquide de lavage (18) est poussé hors du réservoir de liquide (16) à travers une buse (24, 52), le cône de vaporisation (58) étant généré par le fait que le liquide de lavage (18) s'écoule à travers la buse (24, 52), **caractérisé en ce que**,
lors d'un dépassement d'une pression limite dans la conduite de gaz sous pression (9), au moins une soupape de surpression (14, 15) est ouverte dans la conduite de gaz sous pression (9) et qu'ainsi le gaz sous pression s'écoule dans l'environnement et la pression est réduite, est de préférence limitée, dans la conduite de gaz sous pression (9).

14. Procédé selon la revendication 13, **caractérisé en ce que**
la pression du gaz est générée par l'évaporation d'un gaz à partir d'une cartouche de gaz liquéfié (4), notamment d'une cartouche de CO₂ (4), où, de préférence, le gaz est liquéfié partiellement dans une chambre d'évaporation (10) avant qu'il ne soit mené en direction de la soupape de surpression (14, 15).

15. Procédé selon la revendication 13 ou la revendication 14, **caractérisé en ce que**
la pression du gaz est limitée avec une soupape de réduction de pression dans la conduite de gaz sous pression (9) et la pression de gaz limitée par la soupape de réduction de pression est menée à travers la conduite de gaz sous pression (9) vers le réservoir de liquide (16) du liquide de lavage (18) médical, où la pression du gaz est de préférence réduite, de préférence limitée, entre la soupape de réduction de pression et le réservoir de liquide (16) lors d'un dépassement de la pression limite.
